Europäisches Patentamt

European Patent Office

Office européen des brevets

(19)

(11) Veröffentlichungsnummer: **0 287 805 B1**

(12) # EUROPÄISCHE PATENTSCHRIFT

(45) Veröffentlichungstag der Patentschrift: **27.05.92**

(21) Anmeldenummer: **88104036.4**

(22) Anmeldetag: **15.03.88**

Die Akte enthält technische Angaben, die nach
dem Eingang der Anmeldung eingereicht wurden
und die nicht in dieser Patentschrift enthalten sind.

(51) Int. Cl.⁵: **A61K 7/00**, A61K 7/06,
A61K 7/50

(54) **Konservierte Haar- und Körperreinigungsmittel sowie Verwendung einer Konservierungsstoff-Kombination.**

(30) Priorität: **24.04.87 DE 3713684**

(43) Veröffentlichungstag der Anmeldung:
**26.10.88 Patentblatt 88/43**

(45) Bekanntmachung des Hinweises auf die
Patenterteilung:
**27.05.92 Patentblatt 92/22**

(84) Benannte Vertragsstaaten:
**AT BE CH DE ES FR GB GR IT LI NL SE**

(56) Entgegenhaltungen:
**DE-A- 2 637 576**
**DE-A- 3 434 885**
**GB-A- 2 042 581**

(73) Patentinhaber: **Wella Aktiengesellschaft**
**Berliner Allee 65**
**W-6100 Darmstadt(DE)**

(72) Erfinder: **Hölzel, Hans**
**Jahnstr. 9**
**W-6101 Fränkisch-Crumbach(DE)**
Erfinder: **Konrad, Eugen**
**Mecklenburger Str. 101**
**W-6100 Darmstadt(DE)**
Erfinder: **Schwarz, Horst**
**Fliederweg 22 D**
**W-6104 Seeheim(DE)**

EP 0 287 805 B1

**Beschreibung**

Haar- und Körperreinigungsmittel enthalten in der Regel einen Konservierungsstoff, welcher die Mittel vor dem Befall durch Mikroorganismen wirksam schützen soll.

Für den Einsatz in Haar- und Körperreinigungsmitteln werden an einen Konservierungsstoff gegensätzliche Anforderungen gestellt. So sollte er einerseits in physiologischer und dermatologischer Hinsicht gut verträglich sein und andererseits über eine gute keimhemmende oder sogar keimtötende Wirkung verfügen. Beide Anforderungen sind meist nur schwer miteinander vereinbar.

Als übliche in Haar- und Körperreinigungsmitteln verwendete Konservierungsstoffe seien beispielsweise Formaldehyd, 5-Brom-5-nitro-1,3-dioxan, p-Hydroxybenzoesäureester, 2,4,4′-Trichlor-2′-hydroxy-diphenylether, 5-Chlor-2-methyl-3-isothiazolon, 2-Methyl-3-isothiazolon und 2-Brom-2-nitropropan-1,3-diol genannt.

In letzter Zeit sind einige Konservierungsstoffe, wie beispielsweise Formaldehyd und 2-Methyl-3-isothiazolon, in den Verdacht geraten, nicht genügend verträglich zu sein. Von konservierend wirkenden Aldehyden und Phenolen ist bekannt, daß diese mit Proteinen reagieren oder mit diesen in denaturierender Weise in Wechselwirkung treten. Bei Aldehyden besteht zudem die Gefahr der Sensibilisierung.

In der Literatur werden Ameisensäure und Benzoesäure bereits als Konservierungsstoffe beschrieben. Allerdings fand die Ameisensäure bislang in Haar- und Körperreinigungsmitteln keine Verwendung. Die Benzoesäure zeigt in niedriger Konzentration alleine keine ausreichende konservierende Wirkung, während in höheren Konzentrationen eine unerwünschte keratolytische Wirkung auftritt. Die Ameisensäure ist eine unangenehm stechend riechende Säure mit stark korrosiver Wirkung. Ein weiterer Grund, warum Ameisensäure nicht als Konservierungsstoff in Haar- und Körperreinigungsmitteln eingesetzt wird, ist auf deren niedrigen pH-Wert zurückzuführen, bei welchem bereits eine Hydrolyse der üblicherweise in Haar- und Körperpflegemitteln als Tenside eingesetzten Alkylethersulfate auftritt.

Es bestand daher die Aufgabe, ein Haar- und Körperreinigungsmittel mit einem Gehalt an einer Konservierungsstoff-Kombination zur Verfügung zu stellen, welche eine gute konservierende Wirkung besitzt und eine bessere Verträglichkeit aufweist als übliche in derartigen Mitteln verwendete Konservierungsstoffe.

Hierzu wurde gefunden, daß ein wasserhaltiges Haar-und Körperreinigungsmittel mit einem Gehalt an 3 bis 50 Gewichtsprozent eines Tensids oder eines Tensidgemisches, dadurch gekennzeichnet, daß es eine Kombination aus

(A) einem physiologisch verträglichen Salz der Ameisensäure,
(B) Benzoesäure oder deren physiologisch verträglichem Salz und
(C) einer physiologisch unbedenklichen anorganischen oder aliphatischen organischen Säure
enthält, die gestellte Aufgabe in hervorragender Weise löst.

Aus der DE-A-3 434 885 ist bekannt, Benzoesäure oder Ameisensäure in Kombination mit Brenztraubensäure als Konservierungsmittel, unter anderem für Kosmetika, einzusetzen.

Aus der GB-A-2 042 581 ist eine wäßrige Olefinsulfonatlösung mit einem Gehalt an Natriumformiat oder Benzoat in Kombination mit einem Monocarboxylat als Lösungsvermittler bekannt. Olefinsulfonate werden im wesentlichen in Geschirrspülmitteln oder Waschmitteln verwendet.

Aus der DE-A-2 637 576 ist ein Mittel zur Vorbehandlung von Haar vor dem Shampoonieren mit einem Gehalt an einer quaternären Ammoniumverbindung und einer Säure, z. B. Ameisensäure, als Lösungsvermittler bekannt. Jenes Mittel enthält weder ein anionisches Tensid noch Ameisensäure in Form ihres Salzes.

Das erfindungsgemäße Mittel wird durch die vorstehend beschriebene Kombination von Konservierungsstoffen sehr gut konserviert und weist eine sehr gute Verträglichkeit auf.

Die Güte der Konservierung der vorstehend beschriebenen Haar- und Körperreinigungsmittel wurde durch den in der Literatur The United States Pharmacopeia, 21. Auflage, (1985), Seite 1151 beschriebenen Konservierungsbelastungstest festgestellt. Hierbei wurden die Haarbehandlungsmittel mit den Mikroorganismen Candida albicans (ATCC Nr. 10231), Aspergillus niger (ATCC Nr. 16404). Escherichia coli (ATCC Nr. 8739), Pseudomonas aeruginosa (ATCC Nr. 9027) und Staphylococcus aureus (ATCC Nr. 6538) verunreinigt und die Entwicklung der Keimzahl über einen Zeitraum von 28 Tagen überwacht.

Während die Verwendung von Natriumformiat oder Benzoesäure jeweils allein, insbesondere bei Befall durch Pilze, wie zum Beispiel Aspergillus niger, nicht zu einer zufriedenstellenden Konservierung der Haar- und Körperreinigungsmittel führt, werden die Mittel durch die Verwendung der vorstehend beschriebene Kombination aus den Konservierungsstoffen (A), (B) und (C) auch gegen Pilze sehr gut konserviert.

Als geeignete physiologisch verträgliche Salze der Ameisensäure (A) seien beispielsweise das Natriumformiat, das Ammoniumformiat, das Kaliumformiat und das Magnesiumformiat genannt, wobei das Natriumformiat bevorzugt ist.

In dem Haar- und Körperreinigungsmittel ist die Komponente (A) in einer Menge von etwa 0,01 bis 2 Gewichtsprozent, vorzugsweise 0,1 bis 1,0 Gewichtsprozent, enthalten.

Die Komponente (B) ist in einer Menge von etwa 0,05 bis 2 Gewichtsprozent, vorzugsweise 0,1 bis 1,0 Gewichtsprozent, in dem erfindungsgemäßen Mittel enthalten, wobei als Salz der Benzoesäure vor allem ein Alkalibenzoat oder das Ammoniumbenzoat, insbesondere das Natriumbenzoat, geeignet ist.

Als physiologisch unbedenkliche aliphatische organische Säure der Komponente (C) kommen vorzugsweise von Amino-oder Halogensubstituenten freie aliphatische organische Säuren mit 2 bis 6 Kohlenstoffatomen, wie zum Beispiel Zitronensäure, Weinsäure, Milchsäure, Adipinsäure, Maleinsäure, Glyoxylsäure, Gluconsäure und Äpfelsäure in Betracht, wobei Zitronensäure bevorzugt ist, während als anorganische Säure der Komponente (C) vorzugsweise Phosphorsäure verwendet wird.

Die Komponente (C) ist in den neuen Mitteln in einer Menge von etwa 0,1 bis 1,5 Gewichtsprozent, vorzugsweise 0,25 bis 0,5 Gewichtsprozent, enthalten.

Mit der Komponente (C) wird der pH-Wert des Haar- und Körperreinigungsmittels eingestellt, welcher bei 2,0 bis 7,0, vorzugsweise 4,8 bis 5,8, liegt.

Das erfindungsgemäße Haar- und Körperreinigungsmittel liegt vorzugsweise in Form einer wäßrigen Lösung oder Emulsion vor und enthält neben der Kombination aus (A), (B) und (C) mindestens ein anionisches, kationisches, nicht-ionisches oder amphoteres Tensid in einer Menge von etwa 3 bis 50 Gewichtsprozent, vorzugsweise 10 bis 25 Gewichtsprozent.

Unter den für das neue Haar- und Körperreinigungsmittel geeigenten Tensiden seien beispielsweise die folgenden genannt:

a) Die anionischen oberflächenaktiven Agenzien, wie beispielsweise die Alkali-, Erdalkali-, Ammonium- oder Alkanolaminsalze von Alkansulfonaten, Alkylsulfaten und Alkylethersulfaten, die $C_{12}$ bis $C_{18}$-Alkyl- und insbesondere $C_{12}$ bis $C_{14}$-Alkyl-Sulfatnatriumsalzeoder -Triethanolaminsalze, die Natrium-oder Triethanolaminsalze von Lauryl- oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl und Stearylalkohol, allein oder im Gemisch; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispielsweise Lauryl-oder Cetylpyridiniumchlorid, die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agenzien wie beispielsweise die Carboxylderivate von Imidazol, die N-Alkylbetaine, die N-Alkylaminobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalzesowie die Fettsäurealkylamidobetaine, beispielsweise Dimethylcarboxymethylenpropylenamidostearat-betain.

Selbstverständlich kann das erfindungsgemäße Mittel neben den genannten Bestandteilen auch übliche kosmetische Zusätze, beispielsweise Parfümöle in einer Menge von etwa 0,5 bis 5,0 Gewichtsprozent, Trübungsmittel, wie zum Beispiel Ethylenglykoldistearat, in einer Menge von etwa 0,5 bis 5,0 Gewichtsprozent, Perlglanzmittel, wie zum Beispiel ein Gemisch aus Fettsäuremonoalkanolamid und Ethylenglykoldistearat, in einer Menge von etwa 1,0 bis 10,0 Gewichtsprozent, Verdickungsmittel, wie beispielsweise Kokosfettsäurediethanolamid, in einer Menge von etwa 0,5 bis 10,0 Gewichtsprozent, Verdünnungsmittel, wie zum Beispiel 1,2-Propylenglykol oder ethoxyliertes Sorbitanmonolaurat, in einer Menge von etwa 0,5 bis 5,0 Gewichtsprozent, Puffersubstanzen, wie beispielsweise Natriumcitrat oder Natriumphosphat, in einer Menge von etwa 0,1 bis 1,0 Gewichtsprozent, Lösungsvermittler, wie zum Beispiel ethoxyliertes, gegebenenfalls hydriertes Rizinusöl, in einer Menge von etwa 0,1 bis 1,0 Gewichtsprozent, sowie Farbstoffe, wie zum Beispiel Fluorescein-Natriumsalz, in einer Menge von etwa 0,1 bis 1,0 Gewichtsprozent, weiterhin haarund hautpf legende Zusätze, wie zum Beispiel Fettsäureester, Fettalkohole, Fettsäureglyceride, ethoxylierte propoxylierte gesättigte Fettalkohole, Cellulosederivate, kationische Cellulosederivate, Chitosan, kationische Chitinderivate, Lanolinderivate, Cholesterin und Pantothensäure in einer Menge von etwa 0,1 bis 10 Gewichtsprozent, außerdem physiologisch verträgliche anorganische Salze, wie zum Beispiel Natriumchlorid, sowie ferner Feuchthaltemittel, Lichtschutzmittel, Antioxidantien, Komplexbildner und Antischuppenwirkstoffe enthalten.

Weitere übliche, für derartige Mittel bekannte Bestandteile, welche in dem neuen Mittel enthalten sein können, sind beispielsweise bei H. Janistyn, "Handbuch der Kosmetika und Riechstoffe", 3. Band, (1973), Seiten 228 - 284 und 442 - 462, K. Schrader, "Grundlagen und Rezepturen der Kosmetika", (1979), Seiten 375 - 401 und 445 - 455 sowie G. A. Nowak, "Die kosmetischen Präparate", (1984), Seiten 452 - 512, beschrieben.

Die nachfolgenden Beispiele sollen den Gegenstand der Erfindung näher erläutern.

**HAAR- UND KÖRPERREINIGUNGSMITTEL**

**Beispiel 1:** Haarreinigungsmittel

| | |
|---|---|
| 0,15 g | Natriumformiat |
| 0,20 g | Benzoesäure |
| 16,00 g | Laurylalkohol-diglykolethersulfat, Natriumsalz (70prozentiges wäßriges Gel) |
| 0,25 g | kationisches Cellulosederivat (z. B. Polymer JR®-400 der Firma Union Carbide Corp.) |
| 0,30 g | Parfümöl |
| 3,80 g | Natriumchlorid |
| 79,30 g | Wasser, vollentsalzt |
| 100,00 g | |

Das obige Haarreinigungsmittel, dessen pH-Wert mit Zitronensäure auf 5,0 bis 5,8 eingestellt wird, ist sehr gut konserviert und hervorragend verträglich.

**Beispiel 2:** Haarreinigungsmittel

| | |
|---|---|
| 0,15 g | Natriumformiat |
| 0,25 g | Natriumbenzoat |
| 16,00 g | Laurylalkohol-diglykolethersulfat, Natriumsalz (70prozentiges wäßriges Gel) |
| 0,25 g | kationisches Cellulosederivat (z. B. Polymer JR®-400 der Firma Union Carbide Corp.) |
| 0,30 g | Parfümöl |
| 3,80 g | Natriumchlorid |
| 79,25 g | Wasser, vollentsalzt |
| 100,00 g | |

Das obige Haarreinigungsmittel, dessen pH-Wert mit Phosphorsäure auf 5,0 bis 5,8 eingestellt wird, ist sehr gut konserviert und gut verträglich.

**Beispiel 3:** Mildes Körperreinigungsmittel

| | |
|---|---|
| 0,15 g | Natriumformiat |
| 0,20 g | Benzoesäure |
| 20,00 g | Laurylalkohol-diglykolethersulfat, Natriumsalz (70prozentiges wäßriges Gel) |
| 5,00 g | Fettsäureamidoalkylbetain |
| 2,00 g | Kokosfettsäurediethanolamid |
| 0,50 g | Parfümöl |
| 4,00 g | Natriumchlorid |
| 68,15 g | Wasser, vollentsalzt |
| 100,00 g | |

Das obige Körperreinigungsmittel, dessen pH-Wert mit Zitronensäure auf 5,0 bis 5,8 eingestellt wird, ist sehr gut konserviert und ausgezeichnet verträglich.

Alle Prozentangaben dieser Anmeldung stellen, soweit nicht anders angegeben, Gewichtsprozent dar.

**Patentansprüche**

1.  Wasserhaltiges Haar- und Körperreinigungsmittel mit einem Gehalt an 3 bis 50 Gewichtsprozent eines Tensids oder eines Tensidgemisches, dadurch gekennzeichnet, daß es eine Kombination aus
    (A) einem physiologisch verträglichen Salz der Ameisensäure,
    (B) Benzoesäure oder deren physiologisch verträglichem Salz und
    (C) einer physiologisch unbedenklichen anorganischen oder aliphatischen organischen Säure
    enthält.

2.  Mittel nach Anspruch 1, dadurch gekennzeichnet, daß die Komponente (A) das Natriumformiat ist.

3.  Mittel nach den Ansprüchen 1 und 2, dadurch gekennzeichnet, daß die Komponente (A) in einer Menge von 0,01 bis 2,0 Gewichtsprozent, vorzugsweise 0,1 bis 1,0 Gewichtsprozent, enthalten ist.

4.  Mittel nach den Ansprüchen 1 bis 3, dadurch gekennzeichnet, daß die Komponente (B) in einer Menge von 0,05 bis 2 Gewichtsprozent, vorzugsweise 0,1 bis 1,0 Gewichtsprozent, enthalten ist.

5.  Mittel nach den Ansprüchen 1 bis 4, dadurch gekennzeichnet, daß es einen pH-Wert von 2,0 bis 7,0, vorzugsweise von 4,8 bis 5,8, aufweist.

6.  Mittel nach einem der Ansprüche 1 bis 5, dadurch gekennzeichnet, daß die Komponente (c) Zitronensäure oder Phosphorsäure ist.

7.  Mittel nach einem der Ansprüche 1 bis 6, dadurch gekennzeichnet, daß die Komponente (C) in einer Menge von 0,1 bis 1,5 Gewichtsprozent enthalten ist.

8.  Mittel nach einem der Ansprüche 1 bis 7, dadurch gekennzeichnet, daß das Tensid ein anionisches Tensid ist.

9.  Mittel nach einem der Ansprüche 1 bis 8, dadurch gekennzeichnet, daß die Komponente (A) in einer Menge von 0,1 bis 1,0 Gewichtsprozent enthalten ist.

10. Mittel nach einem der Ansprüche 1 bis 9, dadurch gekennzeichnet, daß die Komponente (B) in einer Menge von 0,1 bis 1,0 Gewichtsprozent enthalten ist.

11. Mittel nach einem der Ansprüche 1 bis 10, dadurch gekennzeichnet, daß es einen pH-Wert von 4,8 bis 5,8 aufweist.

12. Mittel nach einem der Ansprüche 1 bis 11, dadurch gekennzeichnet, daß die Komponente (C) in einer Menge von 0,25 bis 0,5 Gewichtsprozent enthalten ist.

13. Verwendung einer Kombination aus
    (A) einem physiologisch verträglichen Salz der Ameisensäure,
    (B) Benzoesäure oder deren physiologisch verträglichem Salz und
    (C) einer physiologisch unbedenklichen anorganischen oder aliphatischen organischen Säure
    zur Konservierung von Haar- und Körperreinigungsmitteln.

**Claims**

1.  Aqueous hair and body cleansing composition containing 3 to 50 weight % of a surfactant or a surfactant mixture, characterised in that it contains a combination of
    (A) a physiologically acceptable salt of formic acid,
    (B) benzoic acid or a physiologically acceptable salt thereof.
    (C) a physiologically harmless inorganic or aliphatic organic acid.

5

**2.** Composition according to Claim 1, characterised in that the component (A) is sodium formate.

**3.** Composition according to Claims 1 and 2, characterised in that the component (A) is present in a quantity of 0.01 to 2.0 weight %.

**4.** Composition according to Claims 1 to 3, characterised in that the component (B) is present in a quantiy of 0,05 to 2 weight %.

**5.** Composition according to Claims 1 to 4, characterised in that it has a pH-value of 2.0 to 7.0.

**6.** Compostion according to Claims 1 to 5, characterised in that the component (C) is citric acid or phosphoric acid.

**7.** Composition according to Claims 1 to 6, characterised in that the component (C) is present in a quantity of 0.1 to 1.5 weight %.

**8.** Compsition according to Claims 1 to 7, characterised in that the surfactant is an anionic surfactant.

**9.** Compostion according to Claims 1 to 8, characterised in that the component (A) is present in a quantity of 0.1 to 1.0 weight %.

**10.** Composition according to Claims 1 to 9, characterised in that the component (B) is present in a quantity of 0.1 to 1.0 weight %.

**11.** Composition according to Claims 1 to 10, characterised in that it has a pH-value of 4,8 to 5,8.

**12.** Compostion according to Claims 1 to 11, characterised that component (C) is present in a quantity of 0,25 to 0,5 weight %.

**13.** Use of a combination of
(A) physiologically acceptable salt of formic acid,
(B) benzoic acid or a physiologically acceptable salt thereof.
(C) a physiologically harmless inorganic or aliphatic organic acid to preserve hair and body cleaning compositions.

**Revendications**

**1.** Produit de nettoyage des cheveux et du corps contenant de l'eau, renfermant 3 à 50 % en poids d'un agent tensio-actif ou d'un mélange d'agents tensio-actifs, caractérisé en ce qu'il renferme une combinaison de:
(A) un sel de l'acide formique physiologiquement compatible,
(B) de l'acide benzoïque ou un sel physiologiquement compatible de celui-ci, et
(C) un acide inorganique ou organique aliphatique physiologiquement sans inconvénients.

**2.** Produit selon la revendication 1, caractérisé en ce que le constituant (A) est le formiate de sodium.

**3.** Produit selon les revendications 1 et 2, caractérisé en ce que le constituant (A) est contenu dans une proportion de 0,01 à 2,0 % en poids.

**4.** Produit selon les revendications 1 à 3, caractérisé en ce que le constituant (B) est contenu dans une proportion de 0,05 à 2 % en poids.

**5.** Produit selon les revendications 1 à 4, caractérisé en ce qu'il présente une valeur de pH de 2,0 a 7,0.

**6.** Produit selon les revendications 1 à 5, caractérisé en ce que le constituant (C) est l'acide citrique ou l'acide phosphorique.

**7.** Produit selon les revendications 1 à 6, caractérisé en ce que le constituant (C) est contenu dans une proportion de 0,1 à 1,5 % en poids.

8. Produit selon les revendications 1 à 7, caractérisé en ce que l'agent tensio-actif est un agent tensio-actif anionique.

9. Produit selon les revendications 1 à 8 caractérisé en ce que le constituant (A) est contenu dans une proportion de 0,1 à 1,0 % en poids.

10. Produit selon les revendications 1 à 9,caractérisé en ce que le constituant est contenu dans une proportion de 0,1 à 1,0 % en poids.

11. Produit selon les revendications 1 à 10, caractérisé en ce qu'il présente une valeur de pH de 4,8 à 5,8.

12. Produit selon les revendications 1 à 11, caractérisé en ce que le constituant (C) est contenu dans une proportion de 0,25 à 0,5 % en poids.

13. Utilisation d'une combinaison de:
    (A) un sel de l'acide formique physiologiquement compatible,
    (a) l'acide benzoïque ou un sel physiologiquement compatible de celui-ci, et
    (C) un acide inorganique ou organique aliphatique physiologiquement sans inconvénients,
    pour la conservation de produits de nettoyage des cheveux et du corps.